# EUROPEAN PATENT APPLICATION

(11) **EP 1 953 121 A1**
(43) Date of publication of application: **06.08.2008**
(21) Application number: 06775585.0
(22) Date of filing: 04.09.2006
(51) Int. Cl.: C02F 11/04, B09B 3/00

(54) **A LIQUID PERMEABLE SILO**

(30) Priority: 26.11.2005 CN 200510022168
(71) Applicant: Ma, Liguo, Chengdu Sichuan 610211 (CN)
(72) Inventor: Ma, Liguo, Chengdu Sichuan 610211 (CN)
(74) Representative: Luchs, Willi
(86) International application number: PCT/CN2006/002268
(87) International publication number: WO 2007/059678

(57) **Abstract**

The liquid-permeable container is made known to the public in this invention. It is used in the process of producing methane from waste such as garbage to ensure continuous and stable operation. The liquid- permeable container is formed by connecting integrated walls with the bottom. The container has a number of openings for separating solids while allowing liquid to pass. Owing to the liquid-permeable container serving as a means of transport in the process of production, waste can be orderly treated in packets, thus ensuring continuous, stable and automatic operation. The production process requires much smaller methane-generating pit, thus substantially reducing the construction cost, maintaining the temperature in the methane-generating pit better, shortening the production cycle and treating larger quantities of waste. During the production process, the soluble, heavy metal-salts and other harmful, soluble substances are kept in the fermentation liquid in the pit, thus discharging less such materials, which is more conducive to the environment protection and can be widely used for disposal of urban waste , sewage, straw, the farm garbage, sewage sludge and so forth.

## Description

### Technical Field

This invention concerns the installations used in the process of producing methane from waste such as garbage, particularly involving a liquid-permeable container that can be loaded with waste.

### Technical Background

Methane is converted from biomass energy. The manual production methods of methane are called anaerobic digestion, that is, the organic matters ferment and decompose under the influence of methane bacteria in the airtight environment. In this way, the feces and refuse are treated positively and turned into valuables. After combustion, the remainders are carbon dioxide and water, causing no pollution to the air, doing no harm to the crops and humanity. Hence, it is a valuable technology of the environment protection. Developing methane resources is also one of the important ways to solve the problem of rural fuels. The raw materials for methane production are all organic matters, such as people and livestock manure, weeds, straw, leaves, litter and so on. Additionally, the raw materials can be the sludge and wastewater containing organic matters from industrial and agricultural production, such as the residue of brew and food processing as well as the sludge from sewage works. There is lots of technology of producing methane such as handling organic waste by anaerobic fermentation and producing methane in the countryside. However, in the conventional production process, the residue in the pit as well as the crusts on the liquid surface in the methane-generating pit leads a great impact on the production of methane, which is a big problem. In addition, in order to ensure the continuity and stability of methane-production, the raw materials for methane in the methane-generating pit are required to be provided balanced. At the same time, because of many kinds of undecomposed matters in the waste, one methane production system needs to handle a variety of pollutants, and the residue must be separated after the production. At present, these problems are not well resolved.

As for the current treatment of organic matters from the industrial and agricultural production as well as urban waste, these ways are usually adopted, that is, first sorting, differentiating the organic matters from the inorganic ones, then incineration, landfill, which are detrimental to the improvement of public sanitation, working conditions and the control of infectious disease. It will be of great importance to the environment protection and the use of renewable sources of energy to treat the biodegradable waste automatically and produce methane from organic waste automatically or semi-automatically on a large scale.

### Invention

The invention provides the liquid-permeable container for continuous and stable production of methane to solve the existing technical problems.

The technological plans involved in this invention are as follows. The liquid-permeable container is formed by connecting integrated walls with the bottom. The container has a number of openings for separating solids while allowing liquid to pass. Load the container with undisposed waste, then put it into the methane-generating pit. Thus the waste is transported and disposed in packets. The fermentation liquid and the fermentation bacteria in the methane-generating pit can freely exchange through the openings, which speeds up the fermentation. During the production process, the soluble, heavy metal-salts and other harmful, soluble substances are kept in the fermentation liquid in the methane-generating pit. The fermented materials will be carried out of the methane-generating pit. Therefore, the continuous and stable waste disposal and methane production can be achieved.

In order to better meet the needs of continuous production, it is by containers that carry the waste in and out of the methane-generating .It is better to make the container box-shaped, with openings set in the walls, facilitating the free exchange between the methane fermentation liquid and the methane fermentation bacteria through openings.

For better fermentation, it is wise to line up the openings evenly along the walls of the box to form a net-like structure.

When trivial silt is used as raw materials, the best way to prevent the residue from spilling out of the loaded container is to cover the liquid-permeable openings with a water-filter layer.

In order to facilitate the continuous operation, a door is set at the bottom of the box for convenient dumping and centralized processing of the remnants after fermentation. To prevent the raw materials from floating and spilling out of the container, a cover is installed at the top of the box.

By the same token, in order to realize the free exchange between the methane fermentation liquid and the methane fermentation bacteria in the methane-generating pit, it is better to use net-like materials to make the cover.

In addition, the container can also be made of weaving and textile materials. The waste in the loaded container and the methane fermentation liquid, the methane fermentation bacteria in the methane-generating pit can exchange freely through the unique pores of the weaving and textile materials.

Similarly, in order to prevent the raw materials from floating and spilling into the methane-generating pit, the top of the container made of weaving materials must be sealed.

As weaving materials are usually flexible, in order to facilitate the delivery and avoid the damage, the railings which are made of rigid materials are set on the periphery of the container.The container is fixed on the railings.

This invention will produce remarkable results. Owing to the liquid-permeable container serving as a means of transport in the process of production, waste can be orderly treated in packets, thus ensuring continuous, stable and automatic operation. The movement of the loaded container stirs the fermentation liquid and accelerates the fermentation. In addition the production process requires much smaller methane-generating pit, thus substantially reducing the construction cost, maintaining the temperature in the pit better, shortening the production cycle and treating larger quantities of waste. During the production process, the soluble, heavy metal-salts and other harmful, soluble substances are kept in the fermentation liquid in pit , thus discharging less such materials, which is more conducive to the environment protection and can be widely used for disposal of urban waste, sewage, straw, farm garbage, sewage sludge and so forth.

### Illustration

- Figure 1: is the front view of the container in the invention, reflecting the external contour of the box-shaped container as well as the openings arranged on the wall.
- Figure 2: is the left view of Figure 1, showing the external contour of the box-shaped container as well as the openings arranged on the wall.
- Figure 3: is the vertical view of Figure 1, showing the external contour of the box-shaped container as well as the cover made of net-like materials.
- Figure 4: is the upward view of Figure 1, showing the external contour of the box-shaped container as well as the door set at the bottom of the container.

### Specific Implementation Methods

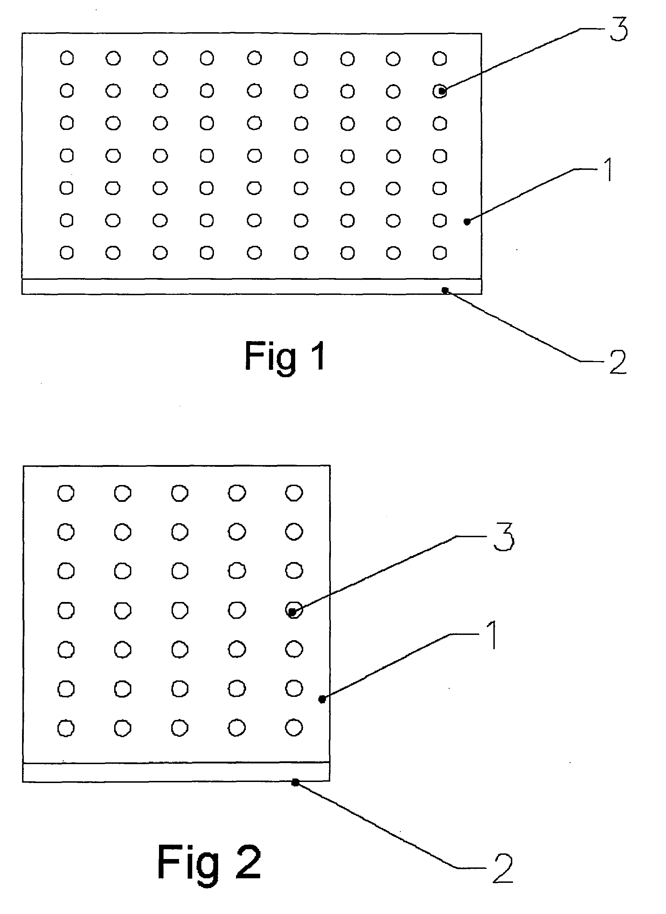

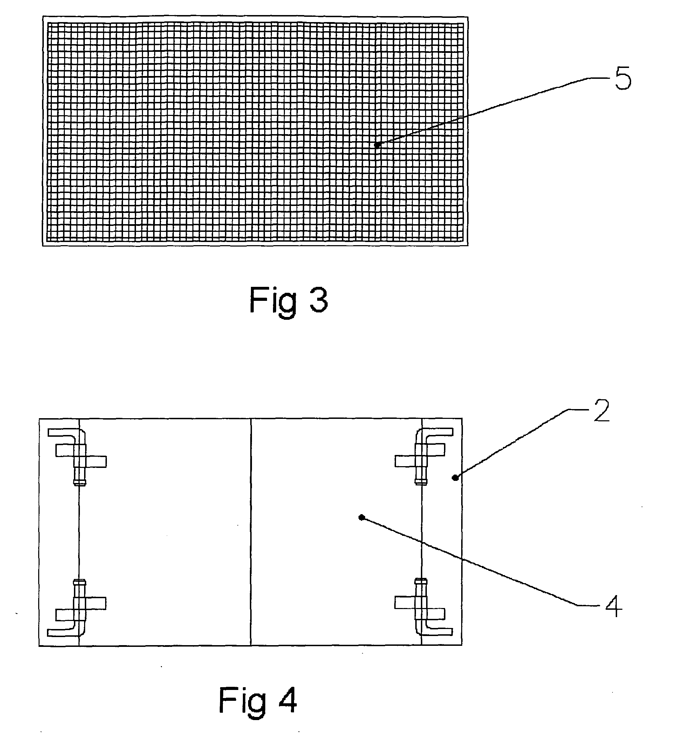

As Figure 1 ~ Figure 5 show, the liquid-permeable container is the tool for delivering and disposing the waste. The liquid-permeable container is formed by connecting integrated walls 1 with the bottom 2, with many openings 3 arranged on the wall to separate the solids while allowing the liquid to pass. Openings 3 can be respectively or simultaneously set on the wall 1 and at the bottom 2 to prevent the waste from spilling, thus achieving the free exchange between the methane fermentation liquid and the methane fermentation bacteria, accelerating waste fermentation.

A loaded container is put into the sealed methane-generating pit filled with water, making sure of sinking the waste into the water. Maintain the temperature at a certain range and collect the methane after a period time of fermentation. After a period of fermentation, take out the container, open it and separate the crude slag from the fine one for further treatment, then put another container with new materials into the pit, which ensures continuous and stable methane production.

To meet better continuous production, the container can be made in form of boxes and the openings 3 are lined up evenly along the walls 1 to form a net-like structure. Therefore, in the whole process, the raw materials are carried into and out of the pit in packets, facilitating the stable production and controlling the production pace.

When trivial silt is used as raw materials, the best way to prevent the residue from spilling out of the container is to cover the liquid-permeable openings 3 with a water-filter layer made of weaving and textile materials etc.. It will not be repeated here.

In order to facilitate the continuous operation, a door 4 is set at the bottom 2 of the box for convenient dumping and centralized processing of the remnants after fermentation.

To prevent the residues from floating and spilling into the methane-generating pit, a cover is installed on top of the box. The cover is made of the net-like materials such as steel or stainless steel wire gauze.

In addition, the container can also be made of weaving and textile materials. The exchange between methane fermentation liquid and the methane fermentation bacteria can freely proceed through the unique openings of the weaving and textile materials, accelerating waste fermentation.

Similarly, in order to prevent the residues from floating and spilling into the methane-generating pit, the top of the container which is made of weaving materials must be sealed.

As weaving materials are usually flexible, in order to facilitate the delivery and avoid the damage, the railings which are made of rigid materials are set on the periphery of the container The container is fixed on the railings. The railings can be made in frame structure, supporting and fixing the container made of the flexible materials.

References in the Figures:
1 wall,
2 bottom,
3 openings,
4 doors,
5 cover.

## Claims

1. Liquid-permeable container is formed by connecting integrated walls (1) with the bottom (2), **characterized by** many openings (3) set on the wall which separate solids while allowing liquid to pass.

2. Container according to claim 1, **characterized by** the box-like shape and openings (3) set on the wall (1).

3. Container according to claim 2, **characterized by** the openings (3) lined up evenly along the wall (1).

4. Container according to claim 3, **characterized by** a water-filter layer covering the openings (3).

5. Container according to claim 1, **characterized by** a door (4) equipped at the bottom (2).

6. Container according to any one of the claims 2, 3 or 4, **characterized by** the cover (5) at the top of the container.

7. Container according to claim 6, **characterized by** the cover (5) made of net-like materials.

8. Container according to claim 1, **characterized by** the container made of weaving and textile materials.

9. Container according to claim 8, **characterized by** sealing the top.

10. Container according to claim 8 or 9, **characterized by** the railings that is made of rigid materials set on the periphery of the container, on which the container is fixed.
